(19) Europäisches Patentamt
European Patent Office
Office européen des brevets

(11) Veröffentlichungsnummer: **0 015 543**
**B1**

(12) **EUROPÄISCHE PATENTSCHRIFT**

(45) Veröffentlichungstag der Patentschrift: **22.06.83**

(21) Anmeldenummer: **80101101.6**

(22) Anmeldetag: **05.03.80**

(51) Int. Cl.³: **C 07 D 215/24**
//(C07D215/24, 215/22)

(54) **8-Oxo-5,6,7,8-tetrahydro-2-chinolon und Verfahren zu dessen Herstellung.**

(30) Priorität: **08.03.79 DE 2909035**

(43) Veröffentlichungstag der Anmeldung:
**17.09.80 Patentblatt 80/19**

(45) Bekanntmachung des Hinweises auf die Patenterteilung:
**22.06.83 Patentblatt 83/25**

(84) Benannte Vertragsstaaten:
**BE CH DE FR GB IT NL**

(56) Entgegenhaltungen:
**DE - A - 2 461 595**
**DE - A - 2 461 604**
**DE - A - 2 461 860**
**DE - A - 2 527 937**
**DE - A - 2 549 889**

(73) Patentinhaber: **HOECHST AKTIENGESELLSCHAFT**
**Postfach 80 03 20**
**D-6230 Frankfurt/Main 80 (DE)**

(72) Erfinder: **Meidert, Helmut, Dr.**
**Griesheimer Stadtweg 11**
**D-6230 Frankfurt am Main 80 (DE)**
Erfinder: **Pressler, Wilfried, Dr.**
**Am Flachsland 15**
**D-6233 Kelkheim (Taunus) (DE)**
Erfinder: **Müller, Werner Heinrich, Dr.**
**Taunusblick 5**
**D-6239 Eppstein/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 015 543 B1

# 8-Oxo-5,6,7,8-tetrahydro-2-chinolon und Verfahren zu dessen Herstellung

Gegenstand dieser Erfindung ist 8-Oxo-5,6,7,8-tetrahydro-2-chinolon der Formel

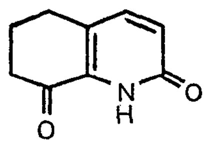

das bisher in der Literatur noch nicht beschrieben worden ist.

Ein weiterer Gegenstand dieser Erfindung ist ein Verfahren zur Herstellung von 8-Oxo-5,6,7,8-tetrahydro-2-chinolon, das dadurch gekennzeichnet ist, daß man 8-Chlor-5,6,7,8-tetrahydro-2-chinolon-hydrochlorid oder 8-brom-5,6,7,8-tetrahydro-2-chinolon-hydrobromid mit Wasser bei 50 bis 100°C zur entsprechenden 8-Hydroxyverbindung hydrolysiert, dann die wäßrige Lösung neutralisiert, aus dem entstandenen 8-Hydroxy-5,6,7,8-tetrahydro-2-chinolon-trihydrat das Kristallwasser entfernt und die wasserfreie Verbindung in einem Lösemittel mit Mangandioxid zum 8-Oxo-5,6,7,8-tetrahydro-2-chinolon dehydriert. Der gesamte Reaktionsablauf wird durch nachstehendes Formelbild veranschaulicht (wobei X=Cl oder Br ist):

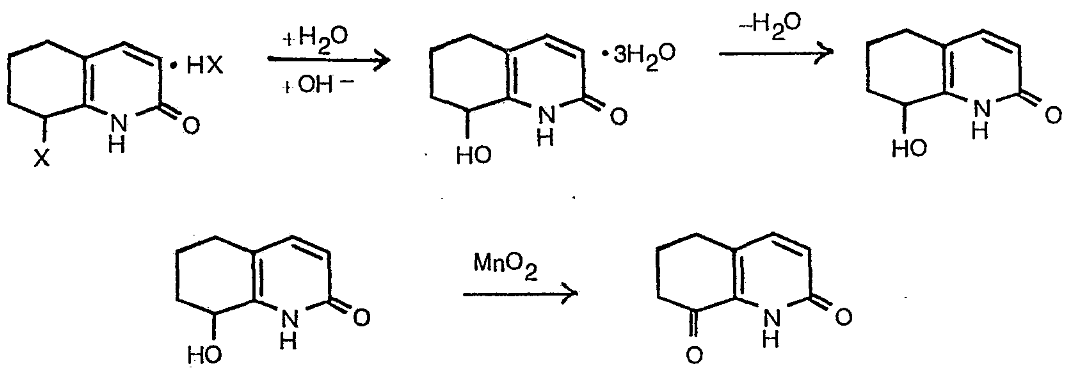


Das erfindungsgemäße 8-Oxo-5,6,7,8-tetrahydro-2-chinolon ist aufgrund seiner Eigenschaften als cyclisches Keton Schlüsselprodukt für eine Vielzahl von Folgeprodukten. Darüberhinaus gelingt es, die erfindungsgemäße Verbindung durch einfache Isomerisierung in das 8-Hydroxy-3,4-dihydro-2-chinolon überzuführen:

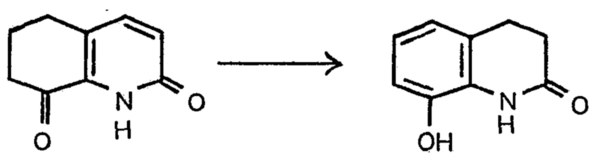

Dieses heterocyclische Phenol kann ein besonderes Interesse beanspruchen, da aromatische Heterocyclen als Kupplungskomponenten für Dispersionsfarbstoffe eine steigende Bedeutung erlangt haben (vgl. z.B. Rys. — M. Zollinger, Leitfaden der Farbstoffchemie, Verlag Chemie 1970, S. 63). Speziell aber ist 8-Hydroxy-3,4-dihydro-2-chinolon aufgrund seines hohen Schmelzpunktes eine hervorragende Kupplungskomponente zur Herstellung von Polyesterfarbstoffen.

Als Ausgangsbasis für die Herstellung des erfindungsgemäßen Ketons dienen 8-Chlor-5,6,7,8-tetrahydro-2-chinolon-hydrochlorid oder 8-brom-5,6,7,8-tetrahydro-2-chinolon-hydrobromid, welche mit der 4 bis 8-fachen Menge Wasser bei 50 bis 100°C, vorzugsweise bei 80 bis 100°C, zum 8-Hydroxy-5,6,7,8-tetrahydro-2-chinolon hydrolysiert werden, das nach Erkalten der — beispielsweise mit Ammoniak — neutralisierten Lösung als Trihydrat in kristalliner Form ausfällt. Für die Herstellung des erfindungsgemäßen Ketons ist es jedoch erforderlich, das wasserfreie 8-Hydroxy-5,6,7,8-tetrahydro-2-chinolon in die Oxydation mit Mangandioxid einzusetzen. Die hierfür notwendige Entwässerung des zunächst entstandenen Trihydrats erfolgt i.allg. durch destillative Entfernung des Hydratwassers als Azeotrop, z.B. mit Butylacetat, Chloroform, Butanol, Toluol oder Cyclohexanon. Von diesen Azeotropbildnern haben sich Butylacetat und Cyclohexanon als besonders geeignet erwiesen, da sie aufgrund eines guten Lösevermögens, ihres relativ hohen Siedepunktes und ihrer minimalen Mischbarkeit mit Wasser eine besonders wirtschaftliche Arbeitsweise gestatten.

Die Dehydrierung des 8-Hydroxy-5,6,7,8-tetrahydro-2-chinolons zum erfindungsgemäßen 8-Oxo-5,6,7,8-tetrahydro-2-chinolon erfolgt mittels Mangandioxid in einem Lösemittel, und zwar i.allg. bei 20 bis 100°C, vorzugsweise bei 30 bis 60°C. Also Lösemittel geeignet sind z.B. Chloroform, Dichlorethan, Butylacetat, Dioxan, Butanol, Cyclohexanon.

Besonders vorteilhaft ist es, Butylacetat, Cyclohexanon oder Chloroform als Azeotropbildner für die Entwässerung des Trihydrats zu verwenden und dann, ohne die wasserfreie Hydroxyverbindung zu isolieren, den Azeotropbildner als Lösemittel für die Dehydrierung der Hydroxyverbindung zu verwenden.

Die als Ausgangsstoffe des erfindungsgemäßen Verfahrens dienenden Verbindungen 8-Chlor-5,6,7,8-tetrahydro-2-chinolon-hydrochlorid und 8-Brom-5,6,7,8-tetrahydro-2-chinolon-hydrobromid werden — wie bereits in der deutschen Patentanmeldung P 28 40 437.7 beschrieben — folgendermaßen hergestellt:

Man setzt 3,4,5,6,7,8-Hexahydro-2-chinolon, das aus Cyclohexanon und Acrylnitril einfach hergestellt werden kann (vgl. J. Org. Chem. *29,* 2781 (1964)), in einem inerten Lösemittel bei Temperaturen zwischen 10 und 50°C mit Chlor bzw. Brom um und erwärmt die Reaktionslösung nach Beendigung der Halogenzugabe auf Temperaturen zwischen 60 und 80°C.

Die Reaktion verläuft gemäß folgender Bruttogleichung (X = Cl bzw. Br):

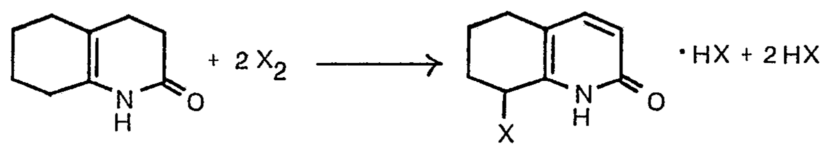


Dabei werden pro Mol Ausgangsverbindung vorzugsweise 1 bis 4 Mol Halogen eingesetzt.

Als Lösemittel für die zu halogenierende Ausgangsverbindung kommen prinzipiell reaktionsinerte Lösemittel, wie chlorierte Kohlenwasserstoffe, Eisessig, Dimethylformamid in Frage, bevorzugt sind chlorierte Kohlenwasserstoffe, wie 1,2-Dichlorethan, Methylenchlorid, Chloroform oder Tetrachlorkohlenstoff.

Die Konzentration der Ausgangsverbindung im Lösemittel wird i.allg. so gewählt, daß ein Gewichtsteil Ausgangsverbindung in 5 bis 12 Volumenteilen des entsprechenden Lösemittels gelöst wird.

Vorzugsweise wählt man pro 1 Mol gelöster Ausgangsverbindung eine Chloreinleit- bzw. Bromeintropfgeschwindigkeit von 0,2 bis 0,6 Mol Halogen pro Stunde. Während hierbei das Chlor im allgemeinen unverdünnt, d.h. ein Chlorstrom direkt in die Lösung der Ausgangsverbindung eingeleitet wird, ist es zweckmäßig, das Brom mit dem zur Anwendung kommenden Lösemittel vorher zu verdünnen.

Während der Nacherwärmung auf 60 bis 80°C scheidet sich das 8-Chlor-5,6,7,8-tetrahydro-2-chinolon-hydrochlorid bzw. das 8-brom-5,6,7,8-tetrahydro-2-chinolon-hydrobromid in kristalliner Form aus der Lösung ab.

Vorzugsweise geht man zur erfindungsgemäßen Herstellung des 8-Oxo-5,6,7,8-tetrahydro-2-chinolons von 8-Chlor-5,6,7,8-tetrahydro-2-chinolon-hydrochlorid aus.

## Beispiel

110 g (0,5 Mol) 8-Chlor-5,6,7,8-tetrahydro-2-chinolon-hydrochlorid werden mit 500 ml Wasser unter Rühren auf ca. 90°C erhitzt, wobei nach kurzer Zeit eine klare Lösung entsteht, die dann mit 75 ml konzentriertem Ammoniak neutralisiert wird. Nach Erkalten der Lösung isoliert man 105 g 8-Hydroxy-5,6,7,8-tetrahydro-2-chinolon-trihydrat, Fp 192°C, entsprechend einer Ausbeute von 95,7% der Theorie.

105 g (0,48 Mol) Trihydrat wird mit 2 l Chloroform unter Rühren erhitzt, das Hydratwasser als Azeotrop entfernt und die Chloroformphase des Destillats dem Rührkolben wieder zugeführt. Nach Beendigung dieser Operation werden der Chloroformlösung insgesamt 350 g Mangandioxid (aktiv gefällt) portionsweise so zugesetzt, daß die Reaktionstemperatur 40°C nicht übersteigt, dann wird noch 3 Stunden bei 40°C intensiv weitergerührt. Anschließend wird vom Mangandioxid abgetrennt, das Chloroform abdestilliert und 65 g hellbraunes 8-Oxo-5,6,7,8-tetrahydro-2-chinolon, entsprechend einer Ausbeute von 83% der Theorie isoliert, das nach Umkristallisation aus Cyclohexanon im geschlossenen Röhrchen bei 253°C schmilzt.

*Versuchsbericht*

a) Isomerisierung von 8-Oxo-5,6,7,8-tetrahydro-2-chinolon zu 8-Hydroxy-3,4-dihydro-2-chinolon

16,3 g (0,1 Mol) 8-Oxo-5,6,7,8-tetrahydro-2-chinolon werden in 375 ml Anisol zusammen mit 5 g Palladium (5%) auf Kohle 30 Minuten auf 150°C erhitzt, die noch heiße Lösung wird sogleich vom Katalysator abfiltriert. Das sich hierbei rasch abscheidende farblose, feinkristalline Isomerisierungsprodukt 8-Hydroxy-3,4-dihydro-2-chinolon entsteht in einer Menge von 12,1 g, entsprechend einer ausbeute von 74,2% der Theorie und schmilzt bei 201°C.

b) Kupplung von 8-Hydroxy-3,4-dihydro-2-chinolon

3,5 g 3-Nitro-anilin werden in 15 ml Eisessig suspendiert und nach Zugabe von 15 ml konzentrierter Salzsäure bei 0°C mit einer Lösung von 2,5 g Natriumnitrit in 5 ml Wasser diazotiert, dann wird diese Lösung mit 25 ml 2-Methoxy-ethanol von 0°C verdünnt.

4,1 g 8-Hydroxy-3,4-dihydro-2-chinolon und 30 g Kaliumacetat werden in 200 ml 2-Methoxy-ethanol gelöst und auf 8 bis 10°C gekühlt, dann wird die Diazoniumlösung langsam hinzugetropft. Nach 10 Minuten weiterem Rühren läßt man die Mischung langsam in einen Liter Wasser einlaufen, saugt den ausgefallenen Niederschlag ab, wäscht und trocknet ihn.

Der entstandene Farbstoff (4,5 g) entspricht der Formel

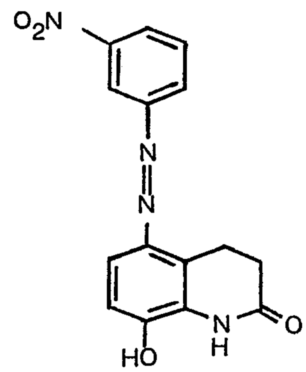

In organischen Lösemitteln, wie z.B. Tetrachlorethylen, löst sich das ockergelbe Produkt mit gelber Farbe. In feine Verteilung gebracht, färbt der Farbstoff synthetische Polyethylenterephthalatgewebe in gelben Farbtönen mit guter Sublimier-, Wasch- und Lichtechtheit.

**Patentansprüche**

1. 8-Oxo-5,6,7,8-tetrahydro-2-chinolon der Formel

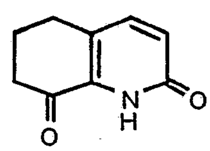

2. Verfahren zur Herstellung von 8-Oxo-5,6,7,8-tetrahydro-2-chinolon, dadurch gekennzeichnet, daß man 8-Chlor-5,6,7,8-tetrahydro-2-chinolon-hydrochlorid oder 8-Brom-5,6,7,8-tetrahydro-2-chinolon-hydrobromid mit Wasser bei 50 bis 100°C zur entsprechenden 8-Hydroxyverbindung hydrolisiert, dann die wäßrige Lösung neutralisiert, aus dem entstandenen 8-Hydroxy-5,6,7,8-tetrahydro-2-chinolon-trihydrat das Kristallwasser entfernt und die wasserfreie Verbindung in enem Lösemittel mit Mangandioxid zum 8-Oxo-5,6,7,8-tetrahydro-2-chinolon dehydriert.

3. Verfahren nach Anspruch 2, dadurch gekennzeichnet, daß man die Hydrolyse bei 80 bis 100°C durchführt.

4. Verfahren nach Anspruch 2 oder 3, dadurch gekennzeichnet, daß man das wasserfreie 8-Hydroxy-5,6,7,8-tetrahydro-2-chinolon aus dessen Trihydrat durch destillative Entfernung des Kristallwassers mit Hilfe eines Azeotropbildners gewinnt.

5. Verfahren nach Anspruch 4, dadurch gekennzeichnet, daß als Azeotropbildner für die Entwässerung und als Lösemittel für die anschließende Dehydrierung des 8-Hydroxy-5,6,7,8-tetrahydro-2-chinolons dieselbe Substanz verwendet wird.

6. Verfahren nach Anspruch 5, dadurch gekennzeichnet, daß als Azeotropbildner und Lösemittel Chloroform, Cyclohexanon oder Butylacetat verwendet werden.

**Revendications**

1. Tétrahydro-5,6,7,8 quinoléine-dione-2,8, corps qui répond à la formule:

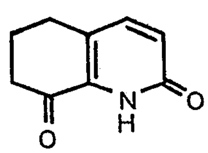

2. Procédé de préparation de la tétrahydro-5,6,7,8 quinoléine-dione-2,8, procédé caractérisé en ce qu'on hydrolyse le chlorhydrate de chloro-8 tétrahydro-5,6,7,8 quinolone-2 ou le bromhydrate de bromo-8 tétrahydro-5,6,7,8 quinolone-2 avec de l'eau à une température de 50—100°C pour obtenir le composé hydroxy-8 correspondant, puis on neutralise la solution aqueuse, on élimine l'eau de cristallisation du trihydrate d'hydroxy-8 tétrahydro-5,6,7,8 quinolone-2 formé et on déshydrogène le composé anhydre, dans un solvant, au moyens du dioxyde de manganèse, de manière à le convertir en la tétrahydro-5,6,7,8 quinoléine-dione-2,8.

3. Procédé selon la revendication 2, caractérisé en ce qu'on effectue l'hydrolyse à une température de 80 à 100°C.

4. Procédé selon l'une des revendications 2 et 3, caractérisé en ce qu'on isole l'hydroxy-8 tétrahydro-5,6,7,8 quinolone-2 anhydre à partir de son trihydrate en chassant l'eau de cristallisation de ce dernier par distillation au moyen d'un générateur d'azéotrope.

5. Procédé selon la revendication 4, caractérisé en ce qu'on utilise la même substance comme générateur d'azéotrope pour la déshydratation et comme solvant pour la déshydrogénation ultérieure de l'hydroxy-8 tétrahydro-5,6,7,8 quinolone-2.

6. Procédé selon la revendication 5, caractérisé en ce qu'on utilise, comme générateur d'azéotrope et solvant, le chloroforme, la cyclohexanone ou l'acétate de butyle.

**Claims**

1. 8-Oxo-5,6,7,8-tetrahydro-2-quinolone of the formula

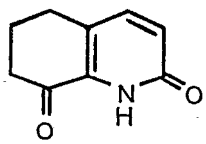

2. A process for the preparation of 8-oxo-5,6,7,8-tetrahydro-2-quinolone, characterized by hydrolizing 8-chloro-5,6,7,8-tetrahydro-2-quinolone hydrochloride or 8-bromo-5,6,7,8-tetrahydro-2-quinolone hydrobromide with water at 50 to 100°C to form the corresponding 8-hydroxy compound, subsequently neutralizing the aqueous solution, removing the water of crystallization from the 8-hydroxy-5,6,7,8-tetrahydro-2-quinolone trihydrate obtained, and dehydro-genating the anhydrous compound in a solvent with manganese dioxide to give 8-oxo-5,6,7,8-tetrahydro-2-quinolone.

3. The process as claimed in claim 2 wherein the hydrolysis is carried out at 80 to 100°C.

4. The process as claimed in claim 2 or 3, characterized by obtaining the anhydrous 8-hydroxy-5,6,7,8-tetrahydro-2-quinolone from the trihydrate thereof of distillation removal of the water of crystallization by means of a water entrainer.

5. The process as claimed in claim 4, characterized by using the same substance as water entrainer for the dehydration and as solvent for the subsequent dehydrogenation of the 8-hydroxy-5,6,7,8-tetrahydro-2-quinolone.

6. The process as claimed in claim 5, characterized by using chloroform, cyclohexanone or butyl acetate as water entrainer and as solvent.